# EUROPEAN PATENT APPLICATION

(11) **EP 3 901 284 A1**
(43) Date of publication of application: **27.10.2021**
(21) Application number: 20170415.2
(22) Date of filing: 20.04.2020
(51) Int. Cl.: C12Q 1/6806, C12Q 1/6848

(54) **CONSTRUCT, KIT AND METHOD FOR SAMPLE TRACKING**

(71) Applicant: BMBK Invest BV, 2320 Hoogstraten (BE)
(72) Inventor: BRASPENNING, Maarten, 2320 Hoogstraten (BE)
(74) Representative: Brantsandpatents bvba

(57) **Abstract**

The current invention relates to a construct, suitable for molecular sample tracking, comprising a unique ID area, wherein said construct further comprises any of the following elements: an exome area, a polyA tail and at least one primer area. The invention relates to a kit comprising a plurality of constructs as well. The invention also relates to a method for molecular marking of a sample.

SEQUENCE LISTING

<110>  BMBK Invest BV

<120>  Construct, kit and method for sample tracking

<130>  BMBK-001-EP

<160>  6

<170>  PatentIn version 3.5

<210>  1
<211>  123
<212>  DNA
<213>  Artificial sequence

<220>
<223>  Nucleic acid construct

<400>  1
atcgatccta atccgctagc cagctggcgc aggctatggg ctgggcggcg gttgagacat      60

cacgacgcag agtggcgtag gtcaaaaaaa aaaaaaaaaa aaaaaaaaaa aaaaaaaaaa     120

aaa                                                                   123

<210>  2
<211>  20
<212>  DNA
<213>  Artificial sequence

<220>
<223>  Nucleic acid construct

<400>  2
atcgatccta atccgctagc                                                  20

<210>  3
<211>  38
<212>  DNA
<213>  Artificial sequence

<220>
<223>  Nucleic acid construct

<400>  3
cagctggcgc aggctatggg ctgggcggcg gttgagac                              38

<210>  4
<211>  8
<212>  DNA
<213>  Artificial sequence

<220>
<223>  Nucleic acid construct

<400>  4
atcacgac                                                                8

<210> 5
<211> 17
<212> DNA
<213> Artificial sequence

<220>
<223> Nucleic acid construct

<400> 5
gcagagtggc gtaggtc 17


<210> 6
<211> 40
<212> DNA
<213> Artificial sequence

<220>
<223> Nucleic acid construct

<400> 6
aaaaaaaaaa aaaaaaaaaa aaaaaaaaaa aaaaaaaaaa 40

## Description

### FIELD OF THE INVENTION

The present invention relates to a construct for molecular sample tracking, comprising a unique ID area. In a second aspect, the present invention also relates to a kit comprising a plurality of said constructs. In a third aspect, the present invention relates to a method for molecular marking of a sample as well.

### BACKGROUND

Sample tracking and identity are essential when processing multiple samples in parallel. Risk of mis-associating study results and samples is incurred at each step of processing, as from the initial sample acquisition. This risk can be partly mitigated by the indirect approach of automated or semi-automated tracking of optically barcoded or otherwise labeled sample containers, however, using this approach, the containers such as vials or tubes are tracked, while the actual sample can still be put in a wrongly labeled container. Tracking errors can potentially be revealed after analysis to verify sample identity, including variant detection at the start and end of the pipeline using arrays or genotyping, bioinformatics comparisons and optical barcoding of samples.

A better solution for sample tracking, is labeling the sample itself in addition to the container. An example thereof is found in the field of next-generation sequencing, where a direct method for tracking sample identity with high effectiveness has been implemented. It relies on unique, known DNA control sequences of approximately 200 bases long, one of which is added directly to each sample, in this case genomic DNA, at the time of receipt. The samples and controls are subsequently inseparable, and the control DNA is processed along with the original sample DNA through all sample preparation, sequencing and analysis steps. The control sequences complement conventional barcode tracking and are used to verify sample identity and cross-contamination on final analysis of sequence data before reporting. The unique DNA sequence can be detected by PCR and/or sequencing at any stage of the process. The control sequences have low identity to any sequence in the National Center for Biotechnology Information non-redundant database (30 bases) and contain no long homopolymer (>7) stretches. This approach is already used in targeted clinical diagnostic whole-genome and RNA-sequencing pipelines and is seen as an inexpensive and flexible solution.

However, there still remains a challenge in making sure the control sequence, also called tracker sequence, can be used in different techniques and is not lost during different sample processing steps prior to sequencing.

The present invention aims to resolve at least some of the problems and disadvantages mentioned above.

### SUMMARY OF THE INVENTION

The present invention and embodiments thereof serve to provide a solution to one or more of above-mentioned disadvantages.

To this end, the present invention relates to a nucleic acid construct for molecular sample tracking according to claim 1. More particular, the nucleic acid construct as described herein comprises a unique ID area and any of the following elements: an exome area, a polyA tail and at least one primer area. Preferred embodiments of the nucleotide construct are shown in any of the claims 2 to 9.

In a second aspect, the present invention relates to a kit comprising a plurality of nucleic acid constructs according to claim 10. A preferred embodiment of the kit is shown in claim 11.

In a third aspect, the present invention relates to a method for molecular marking of a sample according to claim 12 and to preferred embodiments according to claims 13-15.

The nucleic acid construct as described herein provides an advantageous effect as it can be used for internal labelling or marking of a sample and improved tracking of the sample, and remains in said sample through different processing steps.

### DESCRIPTION OF FIGURES

The following description of the figures of specific embodiments of the invention is merely exemplary in nature and is not intended to limit the present teachings, their application or uses. Throughout the drawings, corresponding reference numerals indicate like or corresponding parts and features.
**Figure 1** shows a schematic representation of nucleic acid constructs according to embodiments of the present invention.
**Figure 2** shows a schematic representation of a nucleic acid construct according to a preferred embodiment of the present invention.

### DETAILED DESCRIPTION OF THE INVENTION

The present invention concerns a nucleic acid construct, a kit and a method for sample tracking.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a construct" refers to one or more than one construct.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The recitation of numerical ranges by endpoints includes all numbers and fractions subsumed within that range, as well as the recited endpoints.

Whereas the terms "one or more" or "at least one", such as one or more or at least one member(s) of a group of members, is clear *per se,* by means of further exemplification, the term encompasses *inter alia* a reference to any one of said members, or to any two or more of said members, such as, e.g., any ≥3, ≥4, ≥5, ≥6 or ≥7 etc. of said members, and up to all said members.

The term "deoxyribonucleic acid" or "DNA" is used to indicate the tread-like chain of nucleotides carrying the genetic instructions which are used in growth, development, functioning and reproduction of all known living organisms and many viruses. In this context reference is made to the terms "gene" and "genome", which are sequences of DNA that contain genetic information and can influence the phenotype of an organism. DNA and ribonucleic acid or RNA are "nucleic acids", which are one of the major types of macromolecules that are essential for all known forms of life. Most DNA molecules consist of two biopolymer strands coiled around each other to form a double helix structure. These two DNA strands are called "polynucleotides" since they are composed of monomer units called "nucleotides", which are composed of one of four nitrogen-containing "nucleobases": "cytosine" (C), "guanine" (G), "adenine" (A) or "thymine" (T), a sugar called "deoxyribose", and a phosphate group.

"Ribonucleic acid" or "RNA" is a polymeric molecule essential in various biological processes like coding, decoding, regulation and expression of genes. Like DNA, RNA is assembled as a chain of nucleotides, but unlike DNA it is more often found in nature as a single-strand folded onto itself, rather than a paired double-strand. RNA comprises "ribose" instead of deoxyribose. Cellular organisms use "messenger RNA" or "mRNA" to convey genetic information through the nucleobases guanine (G), "uracil" (U), adenine (A) and cytosine (C) for the synthesis of specific proteins. Other forms of RNA consist of "ribosomal RNA" or "rRNA", which is contained inside the ribosomes and is essential for protein synthesis in all living organisms, and "transfer RNA" or "tRNA", which is an adaptor molecule composed of RNA that serves as the physical link between the mRNA and the amino acid sequence of proteins, by carrying amino acids into the ribosomes. Both prokaryotic and eukaryotic ribosomes can be broken down into two subunits, where "16S" or "16 Svedberg" subunits are typical for prokaryotic organisms and "18S" or "18 Svedberg" subunits are typical for eukaryotic organisms. The 16S rRNA gene and the 18S rRNA gene both comprise variable regions and conserved regions, the latter referring to sequence regions which have undergone very little sequence divergence throughout the evolutionary process. Based on 16S or 18S rRNA sequence information, it is possible to determine the position of the organism in the evolutionary tree, thus identifying the organism.

A "plasmid" is a small DNA molecule within a cell that is physically separated from a chromosomal DNA and can replicate independently. They are most commonly found as small circular, double-stranded DNA molecules in bacteria, however, plasmids are sometimes present in archaea and eukaryotic organisms.

The term "primer" refers to a short strand of RNA or DNA that serves as a starting point for DNA synthesis. It is required for DNA replication because the enzymes that catalyze this process, "DNA polymerases", can only add new nucleotides to an existing strand of DNA. The polymerase starts replication at the 3'-end of the primer, and copies the opposite strand. In order to copy both strands, both a "forward primer" and a "reverse primer" are needed.

With the term "nucleic acid construct", a DNA or RNA molecule is meant, which is specifically designed for the purpose of the current invention. The term "tracker", "tracker sequence" or "tracker construct" as used in this document, equally refers to this nucleic acid construct, unless the context clearly dictates differently. In the context of the present invention, "molecular sample tracking" is used to describe the process of tracing back a sample using molecular techniques or constructs. This process has the purpose of identifying a certain sample in a group of unknown samples.

When referring to a "unique ID area" a unique DNA or RNA sequence is meant, suitable for the identification of a certain sample in a group of unknown samples.

The "exome area" or "exome" is the part of the genome formed by "exons", which are the sequences remaining within the mature RNA after transcription and removal of introns by RNA splicing. It thus comprises all DNA that is transcribed into mature RNA, or also, all of the protein-coding genes in a genome.

The "polyA tail" consists of multiple adenosine monophosphates, or also, it is a stretch of RNA that has only adenine bases. PolyA tails are added in the process of "polyadenylation", which begins as the transcription of a gene terminates. This polyA tail is important for the nuclear export, translation and stability of mRNA.

Reference is made to the term "spike-in", which describes the controlled addition of a product, in this case a nucleic acid construct, in a mixture or sample.

The term "sequencing" refers to the process of determining the precise order of nucleotides within a DNA, RNA or equivalent molecule. The advent of rapid DNA sequencing methods has greatly accelerated biological and medical research and discovery.

"RNA sequencing" is used to analyze the continuously changing cellular transcriptome. In addition to mRNA transcripts, RNA sequencing can also target different populations of RNA, including total RNA and small RNA such as miRNA and tRNA. Specifically,

reference is made to "mRNA sequencing", "total RNA sequencing". "MicroRNA sequencing" or "miRNA sequencing" is a type of RNA sequencing, which differs from other forms of RNA sequencing in that the input material is often enriched for small RNAs. miRNA sequencing is especially useful for examining tissue-specific expression patterns and disease associations. Ribosomal profiling includes, among other types, "16S rRNA sequencing" and "18S rRNA sequencing". Total RNA contains greater than 80% rRNA. A technique called "rRNA depletion" depletes RNA from rRNA, as to enrich non-rRNA.

The term "whole genome bisulfite sequencing" or "WGBS" refers to a sequencing technology used to determine the DNA methylation status of single cytosine molecules by treating the DNA with sodium bisulfite before sequencing. "Reduced representation bisulfite sequencing" or "RRBS" is an efficient and high-throughput technique used to analyze the genome-wide methylation profile on a single nucleotide level. This technique combines restriction enzymes and bisulfite sequencing in order to enrich for the areas of the genome that have a high CpG content.

Reference is made to "shotgun sequencing", which is a method used for sequencing long DNA strands. In shotgun sequencing, DNA is broken up randomly into numerous small segments, which are subsequently sequenced. By performing several rounds of fragmentation and sequencing, computer programs are able to use overlapping ends of different rounds to assemble them into a continuous sequence.

"Microwell plates", "multi-well plates" or "microtiter plates" are flat plates with multiple wells which are used as small test tubes. The microwell plate has become a standard tool in analytical research and clinical diagnostic testing laboratories. A microwell plate typically has 6, 12, 24, 48, 96, 384 or 1536 sample wells arranged in a 2:3 rectangular matrix. Other sizes and dimensions are evenly possible, as known and understood by a person skilled in the art.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the invention provides a nucleic acid construct, suitable for molecular sample tracking, comprising a unique ID area, wherein said nucleic acid construct further comprises any of the following elements: an exome area, a polyA tail and at least one primer area.

With "unique ID area", as mentioned before, is meant a unique DNA or RNA sequence, suitable for the identification of a certain sample in a group of unknown samples.

When adding or spiking said nucleic acid construct to a sample, the different elements will each serve a purpose in making sure the construct is not lost during sample processing steps prior to sequencing. So will the exome area, comprising a sequence from the human genome, remain in the sample after exome capture.

"Exome capture" is a method used to extract and sequence the exome (collection of all exons) in a genome and compare this variation across a sample of individual organisms. This allows studies to quickly focus on the small percent of the genome that is most likely to contain variation that strongly affects phenotypes of interest and/or to identify rates of codon evolution between a set of species to infer the effects of mutation and selection among genes.

The polyA tail, composed of a sequence of adenosine monophosphates, keeps the nucleic acid construct or tracker in the sample after polyA capture.

"PolyA capture" is a method in which RNA is mixed with polyT oligomers, composed of a sequence of thymidine monophosphate, which are mostly covalently attached to a substrate such as magnetic beads. PolyA and polyT are complementary and will therefore 'stick together' or hybridize. As only coding RNA comprises a polyA tail, this method will capture coding RNA and ignore noncoding RNA.

The primer area is any (random) the nucleic acid sequence for which a primer can be designed to which said primer can bind. At the 3'-end of the primer, DNA polymerases can start the replication process. A primer area is required in order to start the sequencing process to analyze which tracker sequence is present in the sample, and thus for molecular identification and tracking of the sample.

In an embodiment, said nucleic acid construct comprises at least two primer areas, preferably at least a forward and a reverse primer area.

Polymerase chain reaction (PCR) is a method used to rapidly make copies of a specific nucleotide sequence. The reaction will amplify a sequence in between a forward and a reverse primer area, to which a forward and reverse primer will bind. If the unique ID area is in between the primer areas, this area will be amplified as well.

The primer areas of the embodiment of present invention, can be any sequence as required in the sample processing steps. If one processing step is a targeted sequencing step, the primer areas of the nucleic acid construct of the invention can be chosen so that these match the primer areas used for this targeted sequencing, and as a result, the sequence in between these primer areas will be amplified and sequenced together with the target sequence for the targeted sequencing. As a non-limiting example, the primer area can have a sequence similar to primer areas designed to target 16S or 18S rRNA sequences. As another example, said primer area can be a T7, T3, CMV or 35S primer area.

Preferably, the unique ID code will be in between the two primer areas, and will thus be amplified for sequencing together with the target sequence. Upon sequencing the target, also the unique ID code will be sequenced, providing information about the sample ID identity.

As mentioned before, the nucleic acid construct of present invention comprises a unique ID area, and further comprises any of the following elements: an exome area, a polyA tail and at least one primer area. The order in which each element is present in the construct, more specifically the ID area, the exome area, the polyA tail and at least one primer area, can be any random order or a specific order chosen in relation to the method, technique, protocol or use for/in which the construct is used.

As can be derived from the definition of "molecular sample tracking", the nucleic acid construct of present invention has the purpose of identifying a certain sample in a group of unknown samples. First of all, the nucleic acid construct of present invention should be added to a sample. This sample can be any sample comprising DNA or RNA. As a result of the molecular marking, the sample can be traced through one or more processing steps.

In an embodiment, a method for molecular marking of a sample comprises adding one or more spike-in constructs to said sample, wherein said construct comprises a unique ID area and further any of the following elements: an exome area, a polyA tail and at least one primer area. Said construct can be a nucleic acid construct of the first aspect of the present invention according to any of the embodiments disclosed in this document.

In an embodiment, the method for molecular marking of a sample comprises the following steps:
a. adding one or more spike-in constructs to said sample;
b. processing said sample through a single or a plurality of processing steps;
c. determining the identity of the spike-in constructs in said processed sample;
d. comparing said identified construct with said originally spiked construct, wherein a match indicates that said sample has been traced through said processing steps.

In an embodiment, determining the identity of the spike-in construct and/or comparing said identified construct can be performed with the help of software. For instance, said method may include sequencing the nucleic acid sample comprising said nucleic acid construct, and determining the sequence of said construct, preferably in a software-based manner.

In a further embodiment, said construct can be a nucleic acid construct of present invention according to any of the embodiments disclosed in this document.

Processing steps can be any processing steps known in the art. Preferably, said processing steps comprise any of the following techniques: DNA sequencing, mRNA sequencing, miRNA sequencing, total RNA sequencing, exome sequencing, whole genome sequencing, whole genome (bisulfite) sequencing (WGBS), 16S rRNA sequencing, 18S rRNA sequencing, shotgun sequencing and reduced representation bisulfite sequencing (RRBS). It will be understood by the skilled person that also other techniques can be used in conjunction with the current construct.

The nucleic acid construct, as disclosed in embodiments of present invention above or below, will remain in the sample through the processing steps by any of the following: polyA capture, rRNA depletion, exome capture, fragmentation and/or size selection or PCR.

In an embodiment, the nucleic acid construct of the present invention is spiked-in in a sample in a concentration of about 0.001-50 %. Preferably the spike-in concentration is 0.005-50 %, more preferably 0.01- 50 %, more preferably 0.05-50 %, more preferably 0.1-50 %, more preferably 0.2-50 %, more preferably 0.3-50 %, more preferably 0.4-50 %, more preferably 0.5-50 %. Preferably the spike-in concentration is 0.005-45 %, more preferably 0.01- 45 %, more preferably 0.05-45 %, more preferably 0.1-45 %, more preferably 0.2-45 %, more preferably 0.3-45 %, more preferably 0.4-45 %, more preferably 0.5-45 %. Preferably the spike-in concentration is 0.005-40 %, more preferably 0.01- 40 %, more preferably 0.05-40 %, more preferably 0.1-40 %, more preferably 0.2-40 %, more preferably 0.3-40 %, more preferably 0.4-40 %, more preferably 0.5-40 %. Preferably the spike-in concentration is 0.005-35 %, more preferably 0.01-35 %, more preferably 0.05-35 %, more preferably 0.1-35 %, more preferably 0.2-35 %, more preferably 0.3-35 %, more preferably 0.4-35 %, more preferably 0.05-35 %. Preferably the spike-in concentration is 0.005-30 %, more preferably 0.01- 30 %, more preferably 0.05-30 %, more preferably 0.1-30 %, more preferably 0.2-30 %, more preferably 0.3-30 %, more preferably 0.4-30 %, more preferably 0.5-30 %. Preferably the spike-in concentration is 0.005-25 %, more preferably 0.01- 25 %, more preferably 0.05-25 %, more preferably 0.1-25 %, more preferably 0.2-25 %, more preferably 0.3-25 %, more preferably 0.4-25 %, more preferably 0.05-25 %. Preferably the spike-in concentration is 0.005-20 %, more preferably 0.01-20 %, more preferably 0.05-20 %, more preferably 0.1-20 %, more preferably 0.2-20 %, more preferably 0.3-20 %, more preferably 0.4-20 %, more preferably 0.5-20 %. Preferably the spike-in concentration is 0.005-15 %, more preferably 0.01-15 %, more preferably 0.05-15 %, more preferably 0.1-15 %, more preferably 0.2-15 %, more preferably 0.3-15 %, more preferably 0.4-15 %, more preferably 0.5-15 %. Preferably the spike-in concentration is 0.005-10 %, more preferably 0.01-10 %, more preferably 0.05-10 %, more preferably 0.1-10 %, more preferably 0.2-10 %, more preferably 0.3-10 %, more preferably 0.4-10 %, more preferably 0.5-10 %. Preferably the spike-in concentration is 0.005-5 %, more preferably 0.01-5 %, more preferably 0.05-5 %, more preferably 0.1-5 %, more preferably 0.2-5 %, more preferably 0.3-5 %, more preferably 0.4-5 %, more preferably 0.5-5 %. Preferably, the spike-in concentration is 0.5-10 %, more preferably 1.0-15 %, more preferably 1.5-15 %, more preferably 2.0-15 %. The spike-in concentration is more preferably 0.5-14 %, more preferably 1.0-14 %, more preferably 1.5-14 %, more preferably 2.0-14 %. The spike-in concentration is more preferably 0.5-13 %, more preferably 1.0-13 %, more preferably 1.5-13 %, more preferably 2.0-13 %. The spike-in concentration is more preferably 0.5-12 %, more preferably 1.0-12 %, more preferably 1.5-12 %, more preferably 2.0-12 %. The spike-in concentration is more preferably 0.5-11 %, more preferably 1.0-11 %, more preferably 1.5-11 %, more preferably 2.0-11 %. The spike-in concentration is more preferably 0.5-10 %, more preferably 1.0-10 %, more preferably 1.5-10 %. The spike-in concentration of the nucleic acid construct in a sample is most preferably about 2.0-10 %.

In an embodiment, no CpG sequences are present in the nucleic acid construct, as these would be subject to change after bisulfite treatment.

In an embodiment, the nucleic acid construct of present invention has a length between 6 and 1000 nucleotides (nt). Preferably the nucleic acid construct has a length of 6-950 nt, more preferably 6-900 nt, more preferably 6-850 nt, more preferably 6-800 nt, more preferably 6-750 nt, more preferably 6-700 nt, more preferably 6-650 nt, more preferably 6-600 nt, more preferably 6-550 nt, more preferably 6-500 nt. The nucleic acid construct more preferably has a length of 10-900 nt, more preferably 15-900 nt, more preferably 20-900 nt, more preferably 25-900 nt, more preferably 30-900 nt, more preferably 35-900 nt, more preferably 40-900 nt, more preferably 45-900 nt, more preferably 50-900 nt, more preferably 55-900 nt, more preferably 60-900 nt, more preferably 65-900 nt, more preferably 70-900 nt. The nucleic acid construct more preferably has a length of 10-800 nt, more preferably 15-800 nt, more preferably 20-800 nt, more preferably 25-800 nt, more preferably 30-800 nt, more preferably 35-800 nt, more preferably 40-800 nt, more preferably 45-800 nt, more preferably 50-800 nt, more preferably 55-800 nt, more preferably 60-800 nt, more preferably 65-800 nt, more preferably 70-800 nt. The nucleic acid construct more preferably has a length of 10-700 nt, more preferably 15-700 nt, more preferably 20-700 nt, more preferably 25-700 nt, more preferably 30-700 nt, more preferably 35-700 nt, more preferably 40-700 nt, more preferably 45-700 nt, more preferably 50-700 nt, more preferably 55-700 nt, more preferably 60-700 nt, more preferably 65-700 nt, more preferably 70-700 nt. The nucleic acid construct more preferably has a length of 10-600 nt, more preferably 15-600 nt, more preferably 20-600 nt, more preferably 25-600 nt, more preferably 30-600 nt, more preferably 35-600 nt, more preferably 40-600 nt, more preferably 45-600 nt, more preferably 50-600 nt, more preferably 55-600 nt, more preferably 60-600 nt, more preferably 65-600 nt, more preferably 70-600 nt. Preferably, the nucleic acid construct has a length of 10-500 nt, more preferably 15-500 nt, more preferably 20-500 nt, more preferably 25-500 nt, more preferably 30-500 nt, more preferably 35-500 nt, more preferably 40-500 nt, more preferably 45-500 nt, more preferably 50-500 nt, more preferably 55-500 nt, more preferably 60-500 nt, more preferably 65-500 nt, more preferably 70-500 nt. The nucleic acid construct more preferably has a length of 6-450 nt, more preferably 10-450 nt, more preferably 15-450 nt, more preferably 20-450 nt, more preferably 25-450 nt, more preferably 30-450 nt, more preferably 35-450 nt, more preferably 40-450 nt, more preferably 45-450 nt, more preferably 50-450 nt, more preferably 55-450 nt, more preferably 60-450 nt, more preferably 65-450 nt, more preferably 70-450 nt. The nucleic acid construct more preferably has a length of 6-400 nt, more preferably 10-400 nt, more preferably 15-400 nt, more preferably 20-400 nt, more preferably 25-400 nt, more preferably 30-400 nt, more preferably 35-400 nt, more preferably 40-400 nt, more preferably 45-400 nt, more preferably 50-400 nt, more preferably 55-400 nt, more preferably 60-400 nt, more preferably 65-400 nt, more preferably 70-400 nt. The nucleic acid construct more preferably has a length of 6-350 nt, more preferably 10-350 nt, more preferably 15-350 nt, more preferably 20-350 nt, more preferably 25-350 nt, more preferably 30-350 nt, more preferably 35-350 nt, more preferably 40-350 nt, more preferably 45-350 nt, more preferably 50-350 nt, more preferably 55-350 nt, more preferably 60-350 nt, more preferably 65-350 nt, more preferably 70-350 nt. The nucleic acid construct more preferably has a length of 6-300 nt, more preferably 10-300 nt, more preferably 15-300 nt, more preferably 20-300 nt, more preferably 25-300 nt, more preferably 30-300 nt, more preferably 35-300 nt, more preferably 40-300 nt, more preferably 45-300 nt, more preferably 50-300 nt, more preferably 55-300 nt, more preferably 60-300 nt, more preferably 65-300 nt, more preferably 70-300 nt. The nucleic acid construct more preferably has a length of 6-275 nt, more preferably 10-275 nt, more preferably 15-275 nt, more preferably 20-275 nt, more preferably 25-275 nt, more preferably 30-275 nt, more preferably 35-275 nt, more preferably 40-275 nt, more preferably 45-275 nt, more preferably 50-275 nt, more preferably 55-275 nt, more preferably 60-275 nt, more preferably 65-275 nt, more preferably 70-275 nt. The nucleic acid construct more preferably has a length of 6-250 nt, more preferably 10-250 nt, more preferably 15-250 nt, more preferably 20-250 nt, more preferably 25-250 nt, more preferably 30-250 nt, more preferably 35-250 nt, more preferably 40-250 nt, more preferably 45-250 nt, more preferably 50-250 nt, more preferably 55-250 nt, more preferably 60-250 nt, more preferably 65-250 nt, more preferably 70-250 nt. The nucleic acid construct more preferably has a length of 6-225 nt, more preferably 10-225 nt, more preferably 15-225 nt, more preferably 20-225 nt, more preferably 25-225 nt, more preferably 30-225 nt, more preferably 35-225 nt, more preferably 40-225 nt, more preferably 45-225 nt, more preferably 50-225 nt, more preferably 55-225 nt, more preferably 60-225 nt, more preferably 65-225 nt, more preferably 70-225 nt. The nucleic acid construct more preferably has a length of 6-200 nt, more preferably 10-200 nt, more preferably 15-200 nt, more preferably 20-200 nt, more preferably 25-200 nt, more preferably 30-200 nt, more preferably 35-200 nt, more preferably 40-200 nt, more preferably 45-200 nt, more preferably 50-200 nt, more preferably 55-200 nt, more preferably 60-200 nt, more preferably 65-200 nt, more preferably 70-200 nt. The nucleic acid construct more preferably has a length of 6-175 nt, more preferably 10-175 nt, more preferably 15-175 nt, more preferably 20-175 nt, more preferably 25-175 nt, more preferably 30-175 nt, more preferably 35-175 nt, more preferably 40-175 nt, more preferably 45-175 nt, more preferably 50-175 nt, more preferably 55-175 nt, more preferably 60-175 nt, more preferably 65-175 nt. Most preferably, the nucleic acid construct has a length of 70-175 nt.

The unique ID area comprised in the nucleic acid construct of present invention has a length of between 4-500 nucleotides. Preferably, the unique ID area has a length of 5-500 nt, more preferably 10-500 nt, more preferably 15-500 nt, more preferably 20-500 nt, more preferably 25-500 nt, more preferably 30-500 nt.

Preferably, the unique ID area has a length of 5-450 nt, more preferably 10-450 nt, more preferably 15-450 nt, more preferably 20-450 nt, more preferably 25-450 nt, more preferably 30-450 nt. Preferably, the unique ID area has a length of 5-400 nt, more preferably 10-400 nt, more preferably 15-400 nt, more preferably 20-400 nt, more preferably 25-400 nt, more preferably 30-400 nt. Preferably, the unique ID area has a length of 5-350 nt, more preferably 10-350 nt, more preferably 15-350 nt, more preferably 20-350 nt, more preferably 25-350 nt, more preferably 30-350 nt. Preferably, the unique ID area has a length of 5-200 nt, more preferably 10-200 nt, more preferably 15-200 nt, more preferably 20-200 nt, more preferably 25-200 nt, more preferably 30-200 nt. Preferably, the unique ID area has a length of 5-150 nt, more preferably 10-150 nt, more preferably 15-150 nt, more preferably 20-150 nt, more preferably 25-150 nt, more preferably 30-150 nt. Preferably, the unique ID area has a length of 5-100 nt, more preferably 10-100 nt, more preferably 15-100 nt, more preferably 20-100 nt, more preferably 25-100 nt, more preferably 30-100 nt. Preferably, the unique ID area has a length of 5-50 nt, more preferably 10-50 nt, more preferably 15-50 nt, more preferably 20-50 nt, more preferably 25-50 nt, more preferably 30-50 nt. Preferably, the unique ID area has a length of 4-20 nt, more preferably 5-20 nt, more preferably 6-20 nt, more preferably 7-20 nt, more preferably 8-20 nt. The unique ID area more preferably has a length of 4-19 nt, more preferably 5-19 nt, more preferably 6-19 nt, more preferably 7-19 nt, more preferably 8-19 nt. The unique ID area more preferably has a length of 4-18 nt, more preferably 5-18 nt, more preferably 6-18 nt, more preferably 7-18 nt, more preferably 8-18 nt. The unique ID area more preferably has a length of 4-17 nt, more preferably 5-17 nt, more preferably 6-17 nt, more preferably 7-17 nt, more preferably 8-17 nt. The unique ID area more preferably has a length of 4-16 nt, more preferably 5-16 nt, more preferably 6-16 nt, more preferably 7-16 nt, more preferably 8-16 nt. The unique ID area more preferably has a length of 4-15 nt, more preferably 5-15 nt, more preferably 6-15 nt, more preferably 7-15 nt. Most preferably, the unique ID area has a length of 8-15 nt.

The exome area comprised in the nucleic acid construct of present invention has a length of between 4 to 500 nucleotides. Preferably, the exome area has a length of 10-500 nt, more preferably 15-500 nt, more preferably 17-500 nt, more preferably 19-500 nt, more preferably 21-500 nt, more preferably 23-500 nt, more preferably 25-500, more preferably 30-500 nt, more preferably 35-500, more preferably 40-500 nt, more preferably 45-500 nt. Preferably, the exome area has a length of 4-450 nt, more preferably 10-450 nt, more preferably 15-450 nt, more preferably 17-450 nt, more preferably 19-450 nt, more preferably 21-40 nt, more preferably 23-450 nt, more preferably 25-450, more preferably 30-450 nt, more preferably 35-450, more preferably 40-450 nt, more preferably 45-450 nt. Preferably, the exome area has a length of 4-400 nt, more preferably 10-400 nt, more preferably 15-400 nt, more preferably 17-400 nt, more preferably 19-400 nt, more preferably 21-400 nt, more preferably 23-400 nt, more preferably 25-400, more preferably 30-400 nt, more preferably 35-400, more preferably 40-400 nt, more preferably 45-400 nt. Preferably, the exome area has a length of 4-350 nt, more preferably 10-350 nt, more preferably 15-350 nt, more preferably 17-350 nt, more preferably 19-350 nt, more preferably 21-350 nt, more preferably 23-350 nt, more preferably 25-350, more preferably 30-350 nt, more preferably 35-350, more preferably 40-350 nt, more preferably 45-350 nt. Preferably, the exome area has a length of 4-300 nt, more preferably 10-300 nt, more preferably 15-300 nt, more preferably 17-300 nt, more preferably 19-300 nt, more preferably 21-300 nt, more preferably 23-300 nt, more preferably 25-300, more preferably 30-300 nt, more preferably 35-300, more preferably 40-300 nt, more preferably 45-300 nt. Preferably, the exome area has a length of 4-250 nt, more preferably 10-250 nt, more preferably 15-250 nt, more preferably 17-250 nt, more preferably 19-250 nt, more preferably 21-250 nt, more preferably 23-250 nt, more preferably 25-250, more preferably 30-250 nt, more preferably 35-250, more preferably 40-250 nt, more preferably 45-250 nt. Preferably, the exome area has a length of 4-200 nt, more preferably 10-200 nt, more preferably 15-200 nt, more preferably 17-200 nt, more preferably 19-200 nt, more preferably 21-200 nt, more preferably 23-200 nt, more preferably 25-200, more preferably 30-200 nt, more preferably 35-200, more preferably 40-200 nt, more preferably 45-200 nt. Preferably, the exome area has a length of 4-150 nt, more preferably 10-150 nt, more preferably 15-150 nt, more preferably 17-150 nt, more preferably 19-150 nt, more preferably 21-150 nt, more preferably 23-150 nt, more preferably 25-150. The exome area more preferably has a length of 4-135 nt, more preferably 10-135 nt, more preferably 15-135 nt, more preferably 17-135 nt, more preferably 19-135 nt, more preferably 21-135 nt, more preferably 23-135 nt, more preferably 25-135 nt. The exome area more preferably has a length of 4-120 nt, more preferably 10-120 nt, more preferably 15-120 nt, more preferably 17-120 nt, more preferably 19-120 nt, more preferably 21-120 nt, more preferably 23-120 nt, more preferably 25-120 nt. The exome area more preferably has a length of 4-105 nt, more preferably 10-105 nt, more preferably 15-105 nt, more preferably 17-105 nt, more preferably 19-105 nt, more preferably 21-105 nt, more preferably 23-105 nt, more preferably 25-105 nt. The exome area more preferably has a length of 4-90 nt, more preferably 10-90 nt, more preferably 15-90 nt, more preferably 17-90 nt, more preferably 19-90 nt, more preferably 21-90 nt, more preferably 23-90 nt, more preferably 25-90 nt. The exome area more preferably has a length of 4-75 nt, more preferably 10-75 nt, more preferably 15-75 nt, more preferably 17-75 nt, more preferably 19-75 nt, more preferably 21-75 nt, more preferably 23-75 nt, more preferably 25-75 nt. The exome area more preferably has a length of 4-60 nt, more preferably 10-60 nt, more preferably 15-60 nt, more preferably 17-60 nt, more preferably 19-60 nt, more preferably 21-60 nt, more preferably 23-60 nt, more preferably 25-60 nt. The exome area more preferably has a length of 4-55 nt, more preferably 10-55 nt, more preferably 15-55 nt, more preferably 17-55 nt, more preferably 19-55 nt, more preferably 21-55 nt, more preferably 23-55 nt, more preferably 25-55 nt. The exome area more preferably has a length of 4-50 nt, more preferably 10-50 nt, more preferably 15-50 nt, more preferably 17-50 nt, more preferably 19-50 nt, more preferably 21-50 nt, more preferably 23-50 nt. Most preferably, the exome area has a length of 25-50 nt.

The polyA tail comprised in the nucleic acid construct of present invention has a length of between 5 and 500 adenosine monophosphates (AMPs). Preferably, the polyA tail has a length of 5-500 AMPs, more preferably 10-500 AMPs, more preferably 15-500 AMPs, more preferably 20-500 AMPs, more preferably 25-500 AMPs, more preferably 30-500 AMPs. Preferably, the polyA tail has a length of 5-450 AMPs, more preferably 10-500 AMPs, more preferably 15-450 AMPs, more preferably 20-450 AMPs, more preferably 25-450 AMPs, more preferably 30-450 AMPs. Preferably, the polyA tail has a length of 5-400 AMPs, more preferably 10-400 AMPs, more preferably 15-400 AMPs, more preferably 20-400 AMPs, more preferably 25-400 AMPs, more preferably 30-400 AMPs. Preferably, the polyA tail has a length of 5-350 AMPs, more preferably 10-350 AMPs, more preferably 15-350 AMPs, more preferably 20-350 AMPs, more preferably 25-350 AMPs, more preferably 30-350 AMPs. Preferably, the polyA tail has a length of 5-300 AMPs, more preferably 10-300 AMPs, more preferably 15-300 AMPs, more preferably 20-300 AMPs, more preferably 25-300 AMPs, more preferably 30-300 AMPs. Preferably, the polyA tail has a length of 5-250 AMPs, more preferably 10-250 AMPs, more preferably 15-250 AMPs, more preferably 20-250 AMPs, more preferably 25-250 AMPs, more preferably 30-250 AMPs. The polyA tail more preferably has a length of 5-225 AMPs, more preferably 10-225 AMPs, more preferably 15-225 AMPs, more preferably 20-225 AMPs, more preferably 25-225 AMPs, more preferably 30-225 AMPs. The polyA tail more preferably has a length of 5-200 AMPs, more preferably 10-200 AMPs, more preferably 15-200 AMPs, more preferably 20-200 AMPs, more preferably 25-200 AMPs, more preferably 30-200 AMPs. The polyA tail more preferably has a length of 5-175 AMPs, more preferably 10-175 AMPs, more preferably 15-175 AMPs, more preferably 20-175 AMPs, more preferably 25-175 AMPs, more preferably 30-175 AMPs. The polyA tail more preferably has a length of 5-150 AMPs, more preferably 10-150 AMPs, more preferably 15-150 AMPs, more preferably 20-150 AMPs, more preferably 25-150 AMPs, more preferably 30-150 AMPs. The polyA tail more preferably has a length of 5-125 AMPs, more preferably 10-125 AMPs, more preferably 15-125 AMPs, more preferably 20-125 AMPs, more preferably 25-125 AMPs, more preferably 30-125 AMPs. The polyA tail more preferably has a length of 5-100 AMPs, more preferably 10-100 AMPs, more preferably 15-100 AMPs, more preferably 20-100 AMPs, more preferably 25-100 AMPs, more preferably 30-100 AMPs. The polyA tail more preferably has a length of 5-75 AMPs, more preferably 10-75 AMPs, more preferably 15-75 AMPs, more preferably 20-75 AMPs, more preferably 25-75 AMPs, more preferably 30-75 AMPs. The polyA tail more preferably has a length of 5-70 AMPs, more preferably 10-70 AMPS, more preferably 15-70 AMPs, more preferably 20-70 AMPs, more preferably 25-70 AMPs, more preferably 30-70 AMPs. The polyA tail more preferably has a length of 5-65 AMPs, more preferably 10-65 AMPs, more preferably 15-65 AMPs, more preferably 20-65 AMPs, more preferably 25-65 AMPs, more preferably 30-65 AMPs. The polyA tail more preferably has a length of 5-60 AMPs, more preferably 10-60 AMPs, more preferably 15-60 AMPs, more preferably 20-60 AMPs, more preferably 25-60 AMPs. Most preferably, the exome area has a length of 30-60 AMPs.

The primer area comprised in the nucleic acid construct of present invention has a length of between 8 and 500 nucleotides. Preferably, the primer area has a length of 10-500 nt, 15-500 nt, more preferably 20-500 nt, more preferably 25-500 nt, more preferably 30-500 nt. Preferably, the primer area has a length of 8-450 nt, more preferably 10-450 nt, 15-450 nt, more preferably 20-450 nt, more preferably 25-450 nt, more preferably 30-450 nt. Preferably, the primer area has a length of 8-400 nt, more preferably 10-400 nt, 15-400 nt, more preferably 20-400 nt, more preferably 25-400 nt, more preferably 30-400 nt. Preferably, the primer area has a length of 8-350 nt, more preferably 10-350 nt, 15-350 nt, more preferably 20-350 nt, more preferably 25-350 nt, more preferably 30-350 nt. Preferably, the primer area has a length of 8-300 nt, more preferably 10-300 nt, 15-300 nt, more preferably 20-300 nt, more preferably 25-300 nt, more preferably 30-300 nt. Preferably, the primer area has a length of 8-250 nt, more preferably 10-250 nt, 15-250 nt, more preferably 20-250 nt, more preferably 25-250 nt, more preferably 30-250 nt. Preferably, the primer area has a length of 8-200 nt, more preferably 10-200 nt, 15-200 nt, more preferably 20-200 nt, more preferably 25-200 nt, more preferably 30-200 nt. Preferably, the primer area has a length of 8-150 nt, more preferably 10-150 nt, 15-150 nt, more preferably 20-150 nt, more preferably 25-150 nt, more preferably 30-150 nt. Preferably, the primer area has a length of 8-100 nt, more preferably 10-100 nt, 15-100 nt, more preferably 20-100 nt, more preferably 25-100 nt, more preferably 30-100 nt. Preferably, the primer area has a length of 8-50 nt, more preferably 9-50 nt, more preferably 10-50 nt, more preferably 11-50 nt, more preferably 12-50 nt, more preferably 13-50 nt, more preferably 14-50 nt, more preferably 15-50 nt. Preferably, the primer area has a length of 8-45 nt, more preferably 9-45 nt, more preferably 10-45 nt, more preferably 11-45 nt, more preferably 12-45 nt, more preferably 13-5 nt, more preferably 14-45 nt, more preferably 15-45 nt. Preferably, the primer area has a length of 8-40 nt, more preferably 9-40 nt, more preferably 10-40 nt, more preferably 11-40 nt, more preferably 12-40 nt, more preferably 13-40 nt, more preferably 14-40 nt, more preferably 15-40 nt. Preferably, the primer area has a length of 8-35 nt, more preferably 9-35 nt, more preferably 10-35 nt, more preferably 11-35 nt, more preferably 12-35 nt, more preferably 13-35 nt, more preferably 14-35 nt, more preferably 15-35 nt. Preferably, the primer area has a length of 8-30 nt, more preferably 9-30 nt, more preferably 10-30 nt, more preferably 11-30 nt, more preferably 12-30 nt, more preferably 13-30 nt, more preferably 14-30 nt, more preferably 15-30 nt. The primer area more preferably has a length of 8-29 nt, more preferably 9-29 nt, more preferably 10-29 nt, more preferably 11-29 nt, more preferably 12-29 nt, more preferably 13-29 nt, more preferably 14-29 nt, more preferably 15-29 nt. The primer area more preferably has a length of 8-28 nt, more preferably 9-28 nt, more preferably 10-28 nt, more preferably 11-28 nt, more preferably 12-28 nt, more preferably 13-28 nt, more preferably 14-28 nt, more preferably 15-28 nt. The primer area more preferably has a length of 8-27 nt, more preferably 9-27 nt, more preferably 10-27 nt, more preferably 11-27 nt, more preferably 12-27 nt, more preferably 13-27 nt, more preferably 14-27 nt, more preferably 15-27 nt. The primer area more preferably has a length of 8-26 nt, more preferably 9-26 nt, more preferably 10-26 nt, more preferably 11-26 nt, more preferably 12-26 nt, more preferably 13-26 nt, more preferably 14-26 nt, more preferably 15-26 nt. The primer area more preferably has a length of 8-25 nt, more preferably 9-25 nt, more preferably 10-25 nt, more preferably 11-25 nt, more preferably 12-25 nt, more preferably 13-25 nt, more preferably 14-25 nt. Most preferably, the primer area has a length of 15-25 nt.

In an embodiment of present invention, the nucleic acid construct comprises an exome area, a polyA tail and at least one primer area. This is advantageous as the same construct can be used in different techniques.

As mentioned before, the order in which each element is present in the construct, more specifically the ID area, the exome area, the polyA tail and at least one primer area, can be any random order or a specific order chosen in relation to the method, technique, protocol or use for/in which the construct is used.

Preferably, the nucleic acid construct comprises an exome area, a polyA tail and at least two primer areas, preferably at least a forward and a reverse primer area.

More preferably, the nucleic acid construct comprises at least and in the exact order a forward primer area, a unique ID area, an exome area, a reverse primer area and a polyA tail.

The advantage of latter embodiment is that the polyA tail is at the end. Hereby, the sequence will be captured during the process of polyA capture. The exome area is in the middle, as it is not required to be at any specific location in order to be picked-up during the exome capture method. The forward and reverse primer areas are located in such manner so that the unique ID area will be amplified during PCR or in a targeted sequencing assay. If the nucleic acid construct is to be used in a targeted sequencing assay, the primer areas will be chosen in such manner that they are composed of the same sequence, or at least a very similar sequence as the primer areas designed especially for the targeted sequencing experiments. As a result, the unique ID area will thus be amplified for sequencing together with a target sequence. Upon sequencing the target, also the unique ID code will be sequenced, giving information about the sample ID identity.

In an embodiment the nucleic acid construct of the invention is a single linear DNA strand. In another embodiment the nucleic acid construct is a plasmid.

The nucleic acid construct of the invention, as described in any embodiment of present invention, can be comprised in a kit, suited for high-throughput processing or screening. Said kit can comprise a plurality of nucleic acid constructs of the present invention. Said kit can further comprise one or more reactants necessary for performing a technique of high-throughput processing or screening. Preferably, it comprises one or more reactants necessary for execution of any of the following techniques: DNA sequencing, mRNA sequencing, miRNA sequencing, total RNA sequencing, exome sequencing, whole genome sequencing, whole genome (bisulfite) sequencing (WGBS), 16S rRNA sequencing, 18S rRNA sequencing, shotgun sequencing and reduced representation bisulfite sequencing (RRBS). In addition, the kit can comprise disposable products used in the executing of techniques, preferably in techniques as described before. Said disposables preferably are a microwell, multi-well or microtiter plate, an Eppendorf tube or an Eppendorf conical tube.

The inclusion in such a kit of a plurality of nucleic acid constructs, of one or more reactants and/or disposables for executing a technique, provides for a ready-to-use lab product, suited for high-throughput processing or screening.

In a second aspect, the invention relates to a kit comprising a plurality of nucleic acid constructs, wherein said nucleic acid construct comprises a unique ID area and any of the following elements: an exome area, a polyA tail and at least one primer area.

Such kit can be a ready-to-use lab product, and can be suited for high-throughput processing or screening. By providing a plurality of nucleic acid constructs through a kit, a plurality of samples can be labelled upon the purchase of or upon acquiring one kit.

In a preferred embodiment, said nucleic acid construct is a nucleic acid construct as described in any of the embodiments as described above.

A person of ordinary skill in the art will appreciate that the kit of the invention can be used as described above in view of the nucleic acid construct of present invention. All features as described in the aspect concerning the nucleic acid construct, as described above as well as below, can relate to any of these aspects, even if they are described in conjunction with a specific aspect.

In an embodiment, said kit further comprises one or more reactants necessary for execution of any of the following techniques: DNA sequencing, mRNA sequencing, miRNA sequencing, total RNA sequencing, exome sequencing, whole genome sequencing, whole genome (bisulfite) sequencing (WGBS), 16S rRNA sequencing, 18S rRNA sequencing, shotgun sequencing and reduced representation bisulfite sequencing (RRBS), and/or disposables such as a microwell, multi-well or microtiter plate, an Eppendorf tube or an Eppendorf conical tube

In a third aspect, the invention relates to a method for molecular marking of a sample comprising DNA or RNA or any mixture thereof such that said sample can be traced through one or more processing steps, wherein one or more spike-in constructs are added to said sample, and wherein said construct comprises a unique ID area and any of the following elements: an exome area, a polyA tail and at least one primer area.

A person of ordinary skill in the art will appreciate that the method of this third aspect of the invention can be used as described above in view of the nucleic acid construct of present invention. Consequently, all aspects of present invention are related. All features as described in the aspect concerning the nucleic acid construct or in the aspect concerning the kit, as described above as well as below, can relate to any of these aspects, even if they are described in conjunction with a specific aspect.

In an embodiment, said construct is a nucleic acid construct as disclosed in any of the embodiments of present invention.

Said method for molecular marking comprises in an embodiment the following:
a. adding one or more spike-in constructs to said sample;
b. processing said sample through a single or a plurality of processing steps;
c. determining the identity of the spike-in constructs in said processed sample;
d. comparing said identified construct with said originally spiked construct, wherein a match indicates that said sample has been traced through said processing steps.

In an embodiment, said spike-in construct is a nucleic acid construct as disclosed in any of the embodiments as described above.

Said processing steps may comprise any of the following techniques: DNA sequencing, mRNA sequencing, miRNA sequencing, total RNA sequencing, exome sequencing, whole genome sequencing, whole genome (bisulfite) sequencing (WGBS), 16S rRNA sequencing, 18S rRNA sequencing, shotgun sequencing and reduced representation bisulfite sequencing (RRBS).

The invention is further described by the following non-limiting examples which further illustrate the invention, and are not intended to, nor should they be interpreted to, limit the scope of the invention.

### DESCRIPTION OF FIGURES

With as a goal illustrating better the properties of the invention the following presents, as an example and limiting in no way other potential applications, a description of a number of preferred representations of the nucleic acid construct according to present invention is provided below, wherein following numbers represent:
1. Unique ID area
2. Exome area
3. PolyA tail
4. Primer area
   4a. Forward primer area
   4b. Reverse primer area

It will be understood by a person skilled in the art that the length of the construct and/or the elements of the construct in the figures are not displayed to scale nor representative for the true length of the construct and/or elements of the construct. The figures are only meant to represent examples of possible combinations of elements and their order in the construct.

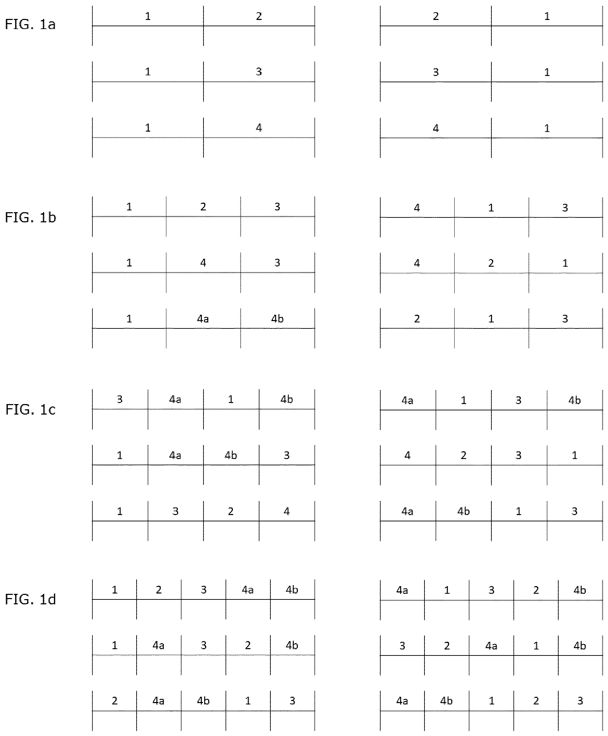

**Figure 1** schematically represents nucleic acid constructs comprising a unique ID area 1 and one **(****Figure 1a****),** two **(****Figure 1b****),** three **(****Figure 1c****)** or four **(****Figure 1d****)** of the following elements: an exome area 2, a polyA tail 3, a primer area 4, a forward primer area 4a and a reverse primer area 4b. The unique ID area 1 and any of the elements (2, 3, 4, 4a, or 4b) can be present in the construct in any order. When such nucleic acid construct is added to or spiked in a sample, the sample can be identified in a group of unknown samples by determining the sequence of the unique ID area 1, even if the sample container is not or wrongly labeled. Each of the elements (2, 3, 4, 4a or 4b) serve a purpose on making sure the nucleic acid construct remains in the sample. So will the exome area 2 be recognized in the exome capture method, while the polyA tail will be recognized in polyA capture. A primer area 4 will be used to start the sequencing process. Both forward 4a and reverse 4b primer areas will ensure a sequence in between said areas will be copied and amplified. Upon sequencing the sample, also the nucleic acid construct comprising the unique ID area 1 will be sequenced, demonstrating the identity of the sample.

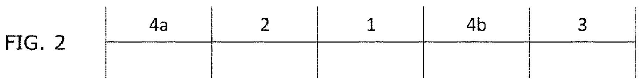

**Figure 2** schematically represents a nucleic acid construct comprising, in the exact order, a forward primer area 4a, a unique ID area 1, an exome area 2, a reverse primer area 4b and a polyA tail 3. As the polyA tail 3 is at the end of the construct, the construct will be captured during the process of polyA capture. The exome area 2 is in the middle, as it is not required to be at any specific location in order to be picked-up during the exome capture method. The forward 4a and reverse 4b primer areas are located in such manner so that the unique ID area 1 and the exome area 2 will be amplified during PCR or in a targeted sequencing assay. If the nucleic acid construct is to be used in a targeted sequencing assay, the primer areas 4 will be chosen in such manner that they are composed of the same sequence, or at least a very similar sequence as the primer areas 4 designed especially for the targeted sequencing experiments. As a result, the unique ID area 1 will thus be amplified for sequencing together with a target sequence. Upon sequencing the target, also the unique ID area 1 will be sequenced, demonstrating the identity of the sample.

### EXAMPLES

The present invention will now be further exemplified with reference to the following example(s). The present invention is in no way limited to the given examples or to the embodiments presented in the figures.

### EXAMPLE: oligo-analysis on nine mixed-species samples

### Introduction

In order to internally label a DNA or RNA sample, a nucleic acid construct was developed which can be used for molecular tracking of a nucleic acid sample. This nucleic acid construct is referred to as a molecular tracker, tracker or tracker sequence. It comprises a unique ID area which will be sequenced in parallel with the DNA or DNA sample when added to this sample, and which will as such exposes its identity, hereby exposing the identity of the sample.

The molecular tracker of this example is a nucleic acid construct comprising different elements in a specific order. More specifically, the nucleic acid construct comprises, in the exact order, a forward primer area, followed by a exome area, a unique ID area, a reverse primer area and a polyA tail. The construct has a total length of 123 nucleotides: the forward primer area consists of 20 nt, the exome area of 38 nt, the unique ID area of 8 nt, the reverse primer area of 17 nt and the polyA tail of 40 AMPs.

More specifically, the nucleic acid construct comprises the following sequence as shown in Table 1:

ATCGATCCTAATCCGCTAGCCAGCTGGCGCAGGCTATGGGCTGGGCGGCGGTTGAGACATCA
CGACGCAGAGTGGCGTAGGTCAAAAAAAAAAAAAAAAAAAAAAAAAAAAAAAAAAAAAAAA

**Table 1: Nucleic acid construct used in Example 1.**

| **Element** | **Length** | **Sequence** |
|---|---|---|
| Forward primer area | 20 nt | ATCGATCCTAATCCGCTAGC |
| Exome area | 38 nt | CAGCTGGCGCAGGCTATGGGCTGGGCGGCGGTTGAGAC |
| Unique ID area | 8 nt | ATCACGAC |
| Reverse primer area | 17 nt | GCAGAGTGGCGTAGGTC |
| PolyA tail | 40 AMPs | AAAAAAAAAAAAAAAAAAAAAAAAAAAAAAAAAAAAAAAA |

The aim of the experiment was to see whether the molecular tracker can be traced back in each sample, and to find out which is the ideal concentration in which the tracker should be added to a DNA or RNA sample in order to be traced back.

For this, three common library prep sequencing protocols were run: RNAseq, reduced representation bisulfite sequencing (RRBS) and whole genome bisulfite sequencing (WGBS). For each protocol, three samples were run with the molecular tracker, spiked-in at the start of the experiment, and this for three different tracker concentrations: 2 %, 5 % and 10 %. As such, nine samples were tested, as shown in Table 2.

**Table 2: samples spiked with molecular tracker in different concentrations for use in a RNAseq, RRBS or WGBS protocol**

| **Sample** | **DNA/RNA** | **Tracker concentration** | **Protocol** |
|---|---|---|---|
| mRNA2 | RNA | 2 % | RNAseq |
| mRNA5 | RNA | 5 % | RNAseq |
| mRNA10 | RNA | 10 % | RNAseq |
| RRBS2 | DNA | 2 % | RRBS |
| RRBS5 | DNA | 5 % | RRBS |
| RRBS10 | DNA | 10 % | RRBS |
| WGBS2 | DNA | 2 % | WGBS |
| WGBS5 | DNA | 5 % | WGBS |
| WGBS10 | DNA | 10 % | WGBS |

The sequencing was loaded on a Miseq pe75. After the sequencing, a bioinformatics approach was used to trace the molecular tracker sequence in the data.

### Bioinformatics approach

### BaseCalling & Demultiplexing

The FASTQ-sequences of the mRNA and WGBS experiments were re-extracted using a custom demultiplexing script using the 'expected indexes' and the unindexed fastq-file. The exact number of reads per experiment can be found in Table 3.

To ensure that only the high quality portion of the data was used for alignments and further downstream analyses and conclusions, QC measures were performed on the reads using Trim Galore v0.4.3. In summary, low-quality base call were trimmed of at the 3' end thereby removing poor quality portions of the reads. In a next step, found adaptor sequences were removed from the 3' end using Cutadapt v1.12. In a final step, trimmed reads with a resulting length < 20bp were filtered out. Final quality assessment was based on the remaining reads using the FastQC quality control tool. The executed quality step can be found in Table 3.

**Table 3: overview of number of reads and quality improving actions per sample**

| **Sample** | **Number of reads** | **Quality adaptations** |
|---|---|---|
| mRNA2 | 1500046 | Adapter removal |
| mRNA5 | 1741629 | Adapter removal |
| mRNA10 | 1565718 | Adapter removal |
| RRBS2 | 770140 | Trim 5' and 3' ends |
| RRBS5 | 571796 | Trim 5' and 3' ends |
| RRBS10 | 623360 | Trim 5' and 3' ends |
| WGBS2 | 3568396 | Trim 5' and 3' ends |
| WGBS5 | 3105953 | Trim 5' and 3' ends |
| WGBS10 | 2600627 | Trim 5' and 3' ends |

### Mapping

For the mRNA-experiments the paired-end 75bp sequence reads were mapped using STAR (v2.5) software to the human reference genome GRCh38. The STAR aligner was used because of its ability to identify splicing-sites. Because of the absence of exon-intron structures in the oligo-sequence, and to avoid splice-site misinterpretations, the mRNA reads were separately mapped to the oligo sequence with the use of the non-splicing Bowtie2 aligner.

For the RRBS and WGBS the standard Bismark mapping procedure was performed (to the GRCz10 and GRCh38 reference genomes respectively). This included a bisulfite conversion step and a Bowtie2 mapping step.

To avoid an overestimation of the reads mapped to the oligo sequence, reads mapped to both genomic sequences and oligo sequence were discarded. Also only reads mapping to the first 90bp (unique sequence + adapters) were considered truly mapping. This to avoid the mapping of general polyA-containing reads and the concurrent expression overestimation.

Read coverage (counts) for all genomic features and 'oligo feature' were calculated using the FeatureCounts script of the SubRead package. The corresponding feature files (GTF) were downloaded from the Ensembl website using a custom script. Read coverage (Table 4) was corrected to the total number of mapped reads per experiment (RPM: reads per million mapped). This in order to be able to compare the coverage results between the experiments.

**Table 4: overview mapping efficiency and oligo coverage per sample**

| **Sample** | **Mapping efficiency** | **Oligo coverage** |
|---|---|---|
| mRNA2 | 91.73 % | 7284 |
| mRNA5 | 91.61 % | 9096 |
| mRNA10 | 87.74 % | 53606 |
| RRBS2 | 43.8 % | 50 |
| RRBS5 | 43.6 % | 24 |
| RRBS10 | 43.8 % | 32 |
| WGBS2 | 75.5 % | 1006 |
| WGBS5 | 74.1 % | 877 |
| WGBS10 | 68.7 % | 773 |

### Results

### Normalized oligo coverage per experiment type

As can be seen in and concluded from Table 4, the tracker could be traced back in all samples, as all samples have oligo coverage.

The results further showed that for the RNAseq and WGBS experiments, the normalized oligo coverage increased with an increasing tracker concentration. The effect of the coverage increase is larger for the WGBS experiment, especially in the 2-5% concentration increase.

Table 4 also points out a low tracker detection for the RRBS experiments, unrelated to the spiked-in tracker concentration. This might be due to the low number of reads sequenced for these experiments, as was shown in Table 3.

### Oligo coverage vs genome coverage

The results further showed that there is no effect of the tracker concentration on the average DNA or RNA sample coverage.

### Conclusions

For each sample of each protocol the molecular tracker could be traced back in the sequencing data. This indicates that the nucleic acid construct used as tracker remains in the sample throughout the processing steps of all used protocols (RNAseq, RRBS and WGBS).

After normalization, the results show that the molecular tracker can be traced back in higher quantities in the sequencing data, when a higher concentration of tracker was spiked-in the sample at the beginning of the experiment.

The results of the experiment also showed that the tracker concentration had no influence of the quality of the sequencing results of the actual DNA or RNA sample, indicating that the molecular tracker can be used without disturbing the acting sequencing results of the DNA or RNA sample.

The present invention is in no way limited to the embodiments described in the examples and/or shown in the figures. On the contrary, methods according to the present invention may be realized in many different ways without departing from the scope of the invention.

## Claims

1. A nucleic acid construct, suitable for molecular sample tracking, comprising a unique ID area **1, characterized in that** said nucleic acid construct further comprises any of the following elements: an exome area **2,** a polyA tail **3** and at least one primer area **4.**

2. Nucleic acid construct according to claim 1, **characterized in that** said nucleic acid construct comprises at least two primer areas **4,** preferably at least a forward **4a** and a reverse primer area **4b.**

3. Nucleic acid construct according to claim 1 or 2, **characterized in that** said nucleic acid construct has a length between 6 and 1000 nucleotides.

4. Nucleic acid construct according to any of the previous claims 1-3, **characterized in that** said unique ID area **1** has a length of between 4 and 500 nucleotides.

5. Nucleic acid construct according to any of the previous claims 1-4, **characterized in that** said exome area **2** has a length of between 5 and 500 nucleotides.

6. Nucleic acid construct according to any of the previous claims 1-5, **characterized in that** said polyA tail **3** has a length of between 5 and 500 adenosine monophosphates.

7. Nucleic acid construct according to any of the previous claims 1-6, **characterized in that** said primer area **4** has a length of between 8 and 500 nucleotides.

8. Nucleic acid construct according to any of the previous claims 1-7, **characterized in that** said nucleic acid construct comprises an exome area **2,** a polyA tail **3** and at least two primer areas **4,** preferably at least a forward **4a** and a reverse primer area **4b.**

9. Nucleic acid construct according to any of the previous claims 1-8, **characterized in that** said nucleic acid construct is a single linear DNA strand or a plasmid.

10. A kit comprising a plurality of nucleic acid constructs according to any of the claims 1-9.

11. Kit according to claim 10, further comprising one or more reactants necessary for execution of any of the following techniques: DNA sequencing, mRNA sequencing, miRNA sequencing, total RNA sequencing, exome sequencing, whole genome sequencing, whole genome (bisulfite) sequencing (WGBS), 16S rRNA sequencing, 18S rRNA sequencing, shotgun sequencing and reduced representation bisulfite sequencing (RRBS) and/or disposables, preferably a microwell, multi-well or microtiter plate, an Eppendorf tube or an Eppendorf conical tube.

12. A method for molecular marking of a sample comprising DNA or RNA or any mixture thereof such that said sample can be traced through one or more processing steps, **characterized in that** one or more spike-in constructs are added to said sample, said construct comprises a unique ID area **1** and any of the following elements: an exome area **2,** a polyA tail **3** and at least one primer area **4.**

13. Method for molecular marking according to claim 12, **characterized in that** said construct is a nucleic acid construct according to any of the claims 1 to 9.

14. Method according to claims 12 or 13, comprising the following:
a. adding one or more spike-in constructs to said sample;
b. processing said sample through a single or a plurality of processing steps;
c. determining the identity of the spike-in constructs in said processed sample;
d. comparing said identified construct with said originally spiked construct, wherein a match indicates that said sample has been traced through said processing steps.

15. Method according to any of the previous claims 12-14, **characterized in that** said processing steps comprise any of the following techniques: DNA sequencing, mRNA sequencing, miRNA sequencing, total RNA sequencing, exome sequencing, whole genome sequencing, whole genome (bisulfite) sequencing (WGBS), 16S rRNA sequencing, 18S rRNA sequencing, shotgun sequencing and reduced representation bisulfite sequencing (RRBS).
